(19) **Europäisches Patentamt / European Patent Office / Office européen des brevets**

(11) **EP 1 584 290 B1**

(12) **EUROPEAN PATENT SPECIFICATION**

(45) Date of publication and mention
of the grant of the patent:
**30.05.2012 Bulletin 2012/22**

(51) Int Cl.:
**A61B 5/053** (2006.01)　　**A61B 5/107** (2006.01)

(21) Application number: **05007432.7**

(22) Date of filing: **05.04.2005**

(54) **Body fat measurement apparatus with a slidable electrode support**

Körperfettmessgerät mit einem verschiebbaren Elektrodenträger

Dispositif de mesure de la graisse corporelle avec un support d'électrodes coulissant

(84) Designated Contracting States:
**DE FR GB**

(30) Priority: **05.04.2004　JP 2004111349**

(43) Date of publication of application:
**12.10.2005 Bulletin 2005/41**

(73) Proprietor: **Tanita Corporation
Tokyo (JP)**

(72) Inventors:
　• **Kasahara, Yasuhiro
　Itabashi-Ku
　Tokyo (JP)**

　• **Honda, Yuka
　Itabashi-Ku
　Tokyo (JP)**
　• **Sunako, Kiharu
　Itabashi-Ku
　Tokyo (JP)**

(74) Representative: **Grünecker, Kinkeldey,
Stockmair & Schwanhäusser
Leopoldstrasse 4
80802 München (DE)**

(56) References cited:
**EP-A- 1 269 917　　EP-A- 1 452 132
EP-A- 1 537 778　　WO-A-02/065900
US-A- 3 955 285**

EP 1 584 290 B1

**Description**

Background of the Invention:

Field of the Invention:

The present invention relates to a body fat measurement apparatus for measuring the impedance of the abdomen of a person under test and for calculating an index of body fat.

Prior Art:

In the past, a body fat measuring apparatus has been developed that comprises impedance measurement electrodes adapted for contact with the hands and feet of a person under test to measure the impedance thereof, on the basis of which an index of body fat is estimated.

Another body fat measurement apparatus for providing body fat data according to a tomogram picture resulted from X-ray CT; Impedance CT and other CT methods or MRI methods have also been developed in order to precisely derive, in particular, a visceral fat area and a subcutaneous fat area for the index of body fat. Another apparatus using the DXA method is additionally known for precisely deriving, in particular, a visceral fat rate and a subcutaneous fat rate for the index of body fat.

Furthermore, various researches and techniques have been disclosed in which impedance measurement electrodes are directly in contact with the surface of the abdomen of a person under test to measure the impedance thereof, on the basis of which an index of body fat is calculated. For example, a study has been done in which a visceral fat mass or a visceral fat area is calculated in correlation with CT methods on the basis of the waist size of a person under test and an impedance value measured using a pair of current supplying electrodes positioned on opposite surfaces, i.e. the front and back sides of an abdomen and a pair of voltage measurement electrodes positioned on both side surfaces of abdomen. (Refer to Non-Patent Document 1.)

Another study has been done in which a belt is wrapped around the abdomen of a person under test in such a manner that impedance measurement electrodes are in contact with the front surface of the abdomen to measure the abdomen's impedance, on the basis of which the subcutaneous fat is estimated. (Refer to Non-Patent Document 2.)

An additional study has been done in which an impedance around an abdomen is measured while changing the part of the body to be measured and the frequency of the electric current supplied thereto, where the part of the body and the frequency suitable for estimation of a visceral fat area are determined. (Refer to Non-Patent Document 3.)

Furthermore, a body fat measurement apparatus has been disclosed in which a visceral fat mass is calculated on the basis of voltage measured using electrodes positioned on an outer peripheral surface of a person under test at an interval that is sufficiently shorter relative to the outer peripheral length of the body of the person and of characteristic amount, reflecting the dimension of the human body represented by e.g. the whole cross section area, the outer peripheral length of the cross section area, or a vertical and a horizontal width of the cross section. (Refer to e.g. Patent Document 1.)

In addition, an impedance measurement apparatus has been disclosed in which a contact surface means on which electrodes are positioned at a fixed interval is in contact with a part of the body of a person under test to measure an impedance thereof. (Refer to e.g. Patent Document 2.)

The document EP 1 269 917 A1 discloses an impedance measurement electrode system with an electrode support adaptable to the lateral width of an abdomen.

Non-Patent Document 1: Literature by Miwa Yana, entitled "Development for Measurement of Visceral Fat Using Abdominal Bioimpedance Method";

Non-Patent Document 2: Literature by Hermann Scharfetter and five others, entitled "Assessing abdominal fatness with local bioimpedance analysis: Basics and experimental findings" ;

Non-Patent Document 3: Literature by Hideaki Komiya and one other, entitled "Research for Estimation of Visceral Fat Area Using Multi-Frequency Impedance Method", Fatness Research, 2003, Vol. 9, No. 1;

Patent Document 1: Japanese Patent No. 3396674; and

Patent Document 2: Patent Application No. 2000-128049.

However, the prior art apparatus designed to estimate an impedance of a trunk portion of a person under test, based on the impedance measured using the electrodes adapted for contact with his/her hands or feet, as described above, is defective in that the reliability of the estimation of an impedance of the trunk portion becomes much lower if the impedance is erroneously measured or cannot be measured at all due to the fact that one of the limbs of the person with which the electrodes are in contact has some trouble, for example.

If the CT or the DXA method is used to derive the index of body fat then the CT method is only effective to measure a body fat area for the index of body fat, wherein the DXA method cannot measure the body fat area. Accordingly, in order to derive the index of body fat including the body fat area, it is necessary to calculate it using at least both the CT and the DXA methods. However, the apparatus using said methods is commonly designed to be operated in the medical field by persons having expert knowledge and techniques, and in addition, the apparatus itself is very expensive. Therefore, it was not common to easily derive the index of body fat.

Furthermore, in the apparatus wherein the impedance measurement electrodes are positionable at opposite sides of the abdomen, either the belt is wrapped around the peripheral part of the abdomen, or the characteristic amount reflecting the dimension of human body is represented by the total cross section area, the outer peripheral length of the cross section area, or the vertical and the horizontal width of the cross section. It is very difficult for a person under test to install the apparatus by himself/herself so that the electrodes or other portions of the apparatus are positioned at the backside of the person, as described above, especially in case where the person under test is confined or almost confined to bed for a long time. Such an installation of the apparatus around the person under test takes much labor even with the assistance by an attendant. In particular, if it is necessary to separately measure the dimension of the part of the body, additional time and labor are necessary, which makes the measurement substantially impractical.

Moreover, in the apparatus wherein the electrodes positioned on the contact surface means are in contact with a part of the body of a person under test to measure the impedance thereof, it tends to produce a difference in the measurement region defined by the electrode contact positions, depending on the dimension of the abdomen of the person, which may cause a variation in the measurement conditions, due to the fact that there is significant personal difference in the distribution of biological tissue such as subcutaneous fat, visceral fat, and muscle, if the measurement is made on the abdomen, not on the limbs of the person under test.

In view of the above, it is an object of the present invention to solve the above-mentioned problems in the prior art and to provide an improved body fat measurement apparatus that allows easy, but precise measurement of an impedance at the front surface of the abdomen of a person under test and that allows a reliable calculation of an index of body fat based on the impedance of the abdomen.

Summary of the Invention:

The present invention provides, a body fat measurement apparatus comprising an impedance measurement electrode system including a pair of current supplying electrodes and a pair of voltage measurement electrodes, those electrodes adapted for contact with a peripheral surface of the abdomen of a person under test to measure an impedance of the abdomen, on the basis of which an index of body fat of the person under test is calculated, said apparatus further comprising: an electrode support unit; an electrode position shift unit; and an arithmetic unit, wherein

said electrode support unit supports the impedance measurement electrode system so that the electrodes thereof can be brought in contact with the front surface of the abdomen of the person under test for measuring an impedance of the abdomen,

said electrode position shift unit move is configured to slidingly the electrode support unit according to the lateral width of the abdomen so that the electrodes of the impedance measurement electrode system are set at suitable positions on the front surface of the abdomen, and

said arithmetic unit is configured to calculate the index of body fat of the person under test on the basis of the impedance of the abdomen.

According to one embodiment of the present invention, said electrode support unit may support the impedance measurement electrode system so that the pair of voltage measurement electrodes is positionable between the pair of current supplying electrodes.

According to another embodiment of the present invention said electrode support unit may include an electrode direction change unit, said electrode direction change unit allowing at least one of the pair of current supplying electrodes and the pair of voltage measurement electrodes to change contact directions in order to suit to the shape of the abdomen.

According to a further embodiment of the present invention, the body fat measurement apparatus may further comprise a frequency switching unit, said frequency switching unit configured to switches frequencies of an AC current supplied to the pair of current supplying electrodes in order to conduct impedance measurements at two or more frequencies of at least one of a higher and a lower frequency range.

According to yet a further embodiment of the present invention, said index of body fat may include at least one of the following: total fat area of the abdomen, subcutaneous fat area, subcutaneous fat thickness, visceral fat area, fat rate of the trunk portion, fat rate of the whole body, and the ratio of subcutaneous fat to visceral fat.

Effects of the Invention:

The present invention is directed toward a body fat measurement apparatus comprising an impedance measurement electrode system including a pair of current supplying electrodes and a pair of voltage measurement electrodes, those electrodes adapted for contact with a peripheral surface of the abdomen of a person under test to measure impedance of the abdomen, on the basis of which an index of body fat of the person under test is calculated. According to the present invention, said apparatus further comprises: an electrode support unit; an electrode position shift unit; and an arithmetic unit, wherein said electrode support unit supports the impedance measurement electrode system so that the electrodes thereof can be brought in contact with the front surface of the abdomen of the person under test for measuring an impedance of the abdomen, said electrode position shift unit configured to slidingly move the electrode support unit according to the lateral width of the abdomen so that the electrodes of the impedance measurement electrode system can be set at suitable positions on the front surface of the abdomen, and said arithmetic unit is configured to calculate the index of body fat of the person under test on the basis of the impedance of the abdomen. Accordingly, by physically moving the electrodes an optimized measurement condition suitable for the abdomen of the person under test can easily be attained, irrespective of the dimensions of the abdomen.

The electrode support unit may support the impedance measurement electrode system so that the pair of voltage measurement electrodes is positioned between the pair of current supplying electrodes. Accordingly, a stable impedance measurement can be attained that is comparable with that of the prior art bioimpedance measurement apparatus for measuring one part of the body, as described earlier.

The electrode support unit may include an electrode direction change unit, said electrode direction change unit allowing at least one of the pair of current supplying electrodes and the pair of voltage measurement electrodes to change contact directions in order to suit the shape of the abdomen of the person under test. Accordingly, the electrodes of the impedance measurement electrode system can closely contact the abdomen of the person under test, which can mitigate any error caused by poor contact conditions of the electrodes.

The body fat measurement apparatus may further comprise a frequency switching unit, said frequency switching unit configured to switche frequencies of an AC current supplied to the pair of current supplying electrodes in order to conduct impedance measurement at two or more frequencies of at least one of a higher and a lower frequency range. Accordingly, it is possible to provide the impedance of a portion of biological tissue such as fat tissue, muscle tissue, at each of a number of frequencies. As the result, measurement at multiple-frequencies can provide the index of body fat with higher precision.

The index of body fat may include at least one of the following: total fat area of the abdomen, subcutaneous fat area, subcutaneous fat thickness, visceral fat area, fat.rate of the trunk portion, fat rate of whole body, and ratio of subcutaneous fat to visceral fat. Therefore, the index of body fat that could previously be measured only by highly expensive apparatus using the CT or the DXA method can, now, easily be measured by an inexpensive apparatus according to the present invention.

Brief Description of the Drawings:

The present invention will be described in more detail with reference to the accompanying drawings, in which:

Fig. 1 is a top view of a body fat measurement apparatus provided as a first example when it is in use;
Fig. 2 is a cross section view of the body fat measurement apparatus taken along the line A-Ain Fig. 1;
Fig. 3 is an electrical block diagram of the body fat measurement apparatus;
Fig. 4 is a model view of the front surface of the abdomen of a person under test modeled by an electrical equivalent circuit of a current path therethrough;
Fig. 5 is a model view of cells illustrating different current paths depending on frequency ranges in electrolyte tissue;
Fig. 6 is a flow chart illustrating the operation of the body fat measurement apparatus;
Fig. 7 is a graph for comparing the total fat area of an abdomen obtained by the CT method with that calculated by the regression equation;
Fig. 8 is a graph for comparing the subcutaneous fat area obtained by the CT method with that calculated by the regression equation;
Fig. 9 is a graph for comparing the visceral fat area obtained by the CT method with that calculated by the regression equation;
Fig. 10 is a graph for comparing the fat rate of the trunk portion obtained by the DXA method with that calculated by the regression equation;
Fig. 11 is a graph for comparing the fat rate of the whole body with the fat rate of the trunk portion;
Fig. 12 is a top view of a body fat measurement apparatus provided as a second example when it is in use;
Fig. 13 is a cross section view of the body fat measurement apparatus taken along the line A-Ain Fig. 12;
Fig. 14 is a top view of a body fat measurement apparatus provided as a third example when it is in use;
Fig. 15 is a cross section view of the body fat measurement apparatus taken along the line A-A in Fig. 14;
Fig. 16 is a top view of a body fat measurement apparatus provided as a fourth example when it is in use;
Fig. 17 is a cross section view of the body fat measurement apparatus taken along the line A-A in Fig. 16;

Fig. 18 is a top view of a body fat measurement apparatus according to a first embodiment of the present invention when it is in use;

Fig. 19 is a cross section view of the body fat measurement apparatus taken along the line A-Ain Fig. 18; and

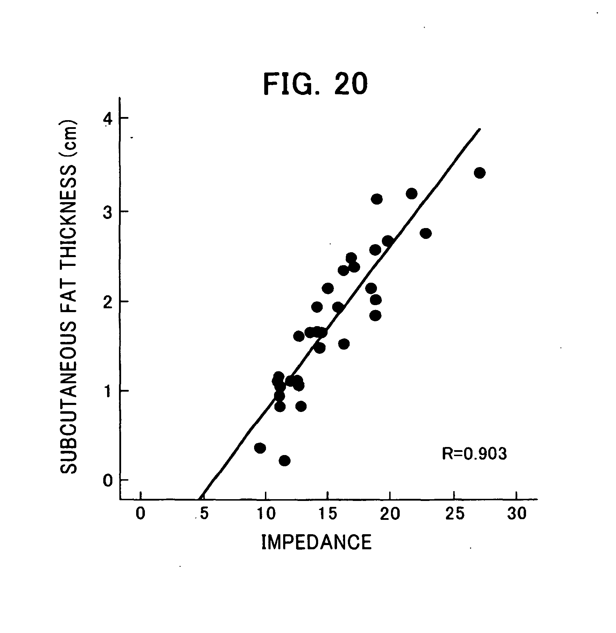

Fig. 20 is a graph illustrating relationship between subcutaneous fat thickness resulted by the CT method and "$Z_{6.25}$".

Best Mode for Embodying the Invention:

.

A body fat measurement apparatus according to the present invention comprises an impedance measurement electrode system including a pair of current supplying electrodes and a pair of voltage measurement electrodes, those electrodes adapted for contact with the peripheral surface of the abdomen of a person under test to measure an impedance of the abdomen, on the basis of which an index of body fat of the person under test is calculated. The apparatus further comprises: an electrode support unit; an electrode position shift unit; and an arithmetic unit, wherein said electrode support unit supports the impedance measurement electrode system so that the electrodes thereof can be brought in contact with the front surface of the abdomen of the person under test for measuring an impedance of the abdomen, said electrode position shift unit configured to slidingly move the electrode support unit according to configured to slidingly lateral width of the abdomen so that the electrodes of the impedance measurement electrode system can be set at suitable positions on the front surface of the abdomen, and said arithmetic unit is configured to calculate the index of body fat of the person under test on the basis of the impedance of the abdomen.

The electrode support unit may support the impedance measurement electrode system so that the pair of voltage measurement electrodes is positioned between the pair of current supplying electrodes.

The electrode support unit may include an electrode direction change unit, said electrode direction change unit allowing at least one of the pair of current supplying electrodes and the pair of voltage measurement electrodes to change contact directions in order to suit the shape of the abdomen of the person under test.

The body fat measurement apparatus may further comprise a frequency switching unit, said frequency switching unit configured to switch frequencies of an AC current supplied to the pair of current supplying electrodes in order to conduct impedance measurements at two or more frequencies of at least one of a higher and a lower frequency range.

The index of body fat may include at least one of the following: total fat area of the abdomen, subcutaneous fat area, subcutaneous fat thickness, visceral fat area, the fat rate of the trunk portion, fat rate of the whole body, and the ratio of subcutaneous fat to visceral fat.

First Example:

A first example discloses a body fat measurement apparatus comprising an electrode support unit having an impedance measurement electrode system consisting of a pair of current supplying electrodes and a pair of voltage measurement electrodes all disposed on a contact surface of the electrode support unit at a fixed interval, and arm portions slidably movable to suit the lateral width of the abdomen of a person under test, wherein the electrodes of the impedance measurement electrode system are positionable on the front surface of abdomen about the center of the abdomen, i.e. a navel position, relative to the lateral width of the abdomen, so that any effect of the dimensions of the impedance measurement region relative to the lateral width of abdomen on the abdomen's impedance is corrected to allow for a plurality of persons under test to be measured in the same measurement conditions. Furthermore, an index of body fat can easily be calculated using a regression equation obtained as the result of the correlation of such a corrected abdomen's impedance with an index of body fat preliminarily derived by the CT or the DXA method. Moreover, the impedance measurement is conducted by switching between two frequency ranges:

a higher frequency range and a lower frequency range. Accordingly, any impedance strongly reflecting muscle tissue or subcutaneous fat tissue in the abdomen can be measured, whereby the index of body fat can be calculated with higher precision.

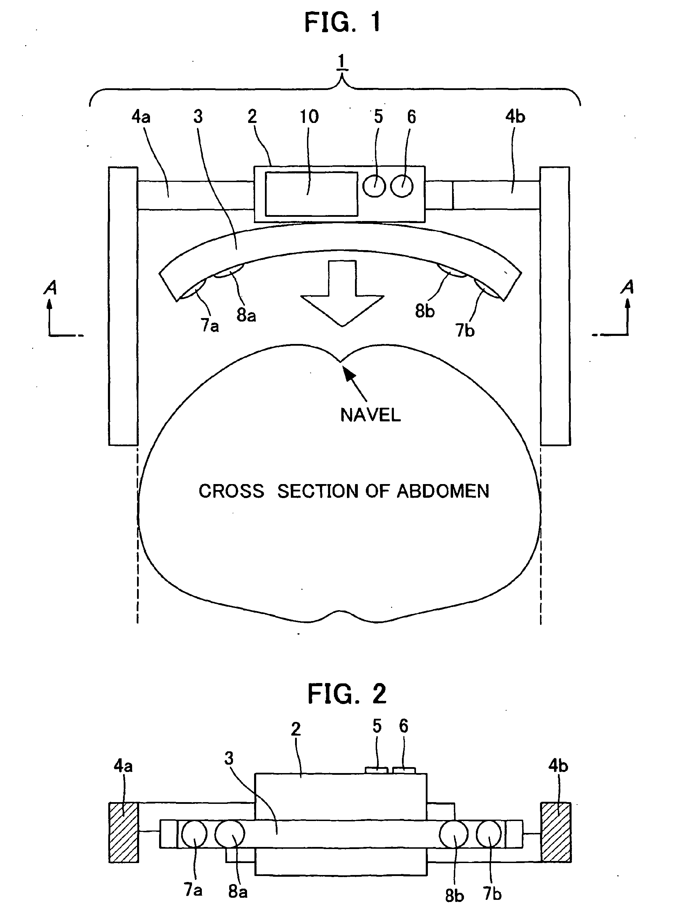

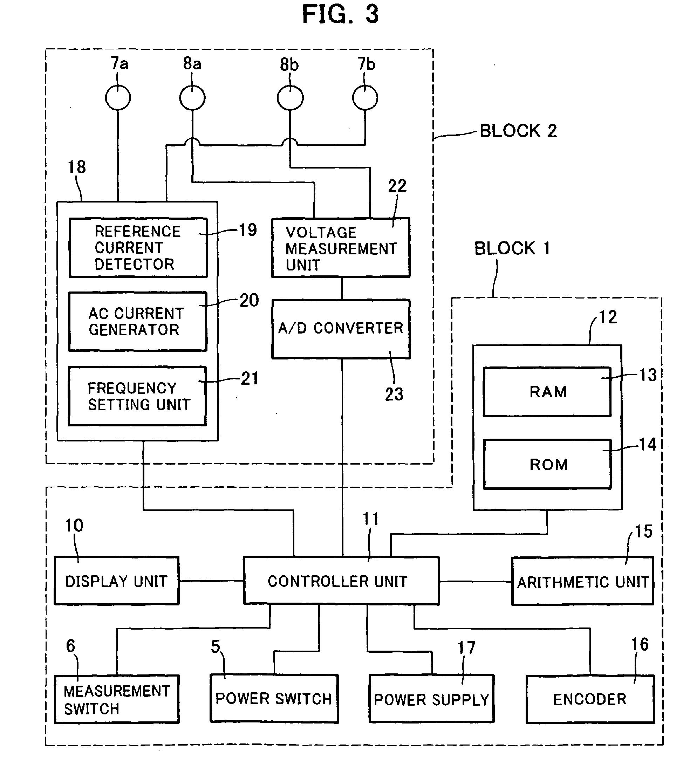

Referring now to Figs. 1 to 3, a body fat measurement apparatus provided as a first example will be described in more detail. In particular, Fig. 1 is a top view of a body fat measurement apparatus when it is in use; Fig. 2 is a cross section view of the body fat measurement apparatus taken along the line A-A in Fig. 1; and Fig. 3 is an electrical block diagram of the body fat measurement apparatus.

As shown in Fig. 1, the body fat measurement apparatus 1 comprises a body portion 2, an electrode support unit 3, and left and right arm portions 4a and 4b.

The body portion 2 of the apparatus 1 includes a power switch 5 for turning the apparatus ON or OFF, a measurement switch 6 for starting a measurement, and a display unit 10 for displaying a guidance message for the measurement and the result of the measurement.

The electrode support unit 3 is designed to be mounted to a side surface of the body portion 2 and is formed from some flexible member to provide a curved leaf spring so that one side thereof that is to contact the front surface of the abdomen of a person under test is directed inwardly, as shown in Fig. 1. An impedance measurement electrode system 9 including a pair of current supplying electrodes 7a and 7b and a pair of voltage measurement electrodes 8a and 8b is provided near both end portions of the electrode support unit 3. In particular, the pair of voltage measurement electrodes 8a and 8b is positioned between the pair of current supplying electrodes 7a and 7b.

Accordingly, when the body fat measurement apparatus 1 is pressed against the front surface of abdomen of the person under test in the direction as indicated by a black arrow in Fig. 1 then the electrode support unit 3 may be flexed to suit the shape of the abdomen of the person under test. As the result, the electrodes of the impedance measurement electrode system 9 can closely contact the front surface of the abdomen of the person under test.

The left and right arm portions 4a and 4b each has a generally "L" shaped construction. One end of the L is inserted into the body portion 2 and is supported by some sliding mechanism (not shown). The sliding mechanism acts to slidably move the left and right arm portions 4a and 4b in the left and right directions respectively at an equal distance from the body portion 2 as the center. Accordingly, simply by making contact between the left and right arm portions 4a and 4b and the left and right sides of the abdomen of the person under test, respectively, the body portion 2 of the apparatus can always be positioned at the center of the front surface of the abdomen of the person under test. As a result, the electrodes of the impedance measurement electrode system 9 can be positioned symmetrically about the center of the abdomen in the left-and-right direction.

Referring now to the electrical block diagram of Fig. 3, the internal configuration of the body fat measurement apparatus 1 will be described. The block diagram generally consists of block 1 and block 2. In particular, block 1 includes functions related to arithmetic operations, data input and output, and storage, and the block 2 includes the function of impedance measurement.

In the block 1 the body fat measurement apparatus 1 includes a controller unit 11 for executing all the control functions of the apparatus. A storage unit 12 and an arithmetic unit 15 are connected to the controller unit 11. The storage unit 12 includes a RAM 13 for temporally storing some measurement data and arithmetic result, and a ROM 14 for storing the control program for the apparatus, calculation equations or a judgment program for the index of body fat that can be set in advance, and the frequency of an AC current supplied in the impedance measurement. The arithmetic unit 15 is configured to calculate the impedance value and the index of body fat.

Also connected with the controller unit 11 are an encoder unit 16 and a power supply 17. The encoder unit 16 acts to measure the distance between the arm portions 4a and 4b by measuring the distance by which the arm portions 4a and 4b have been moved by the sliding mechanism of the body portion 2. That is to say, a side-to-side distance across the abdomen represented by the distance between the arm portions 4a and 4b (hereafter referred to as "lateral width of abdomen") is automatically measured by the encoder unit 16.

Furthermore, the power switch 5, the measurement switch 6 and the display unit 10 are connected to the controller unit 11.

In block 2 the current supplying electrodes 7a and 7b are connected to a current supply unit 18, and the voltage measurement electrodes 8a and 8b are connected to a voltage measurement unit 22 which is then connected to an A/D converter 23. Finally, both are connected to the controller unit 11 in the block 1.

The current supply unit 18 includes a reference current detector 19, an AC current generator 20, and a frequency setting unit 21. The controller unit 11 controls the frequency setting unit 21 to set the frequency to the predetermined one, on the basis of which the AC current generator 20 generates an AC current. This AC current is detected in the reference current detector 19 as the current to be supplied to the person under test, and then, it is supplied to the current supplying electrodes 7a and 7b.

An analog voltage signal picked up by the voltage measurement electrodes 8a and 8b is measured in the voltage measurement unit 22, and then, it is converted to a digital signal in the A/D converter 23.

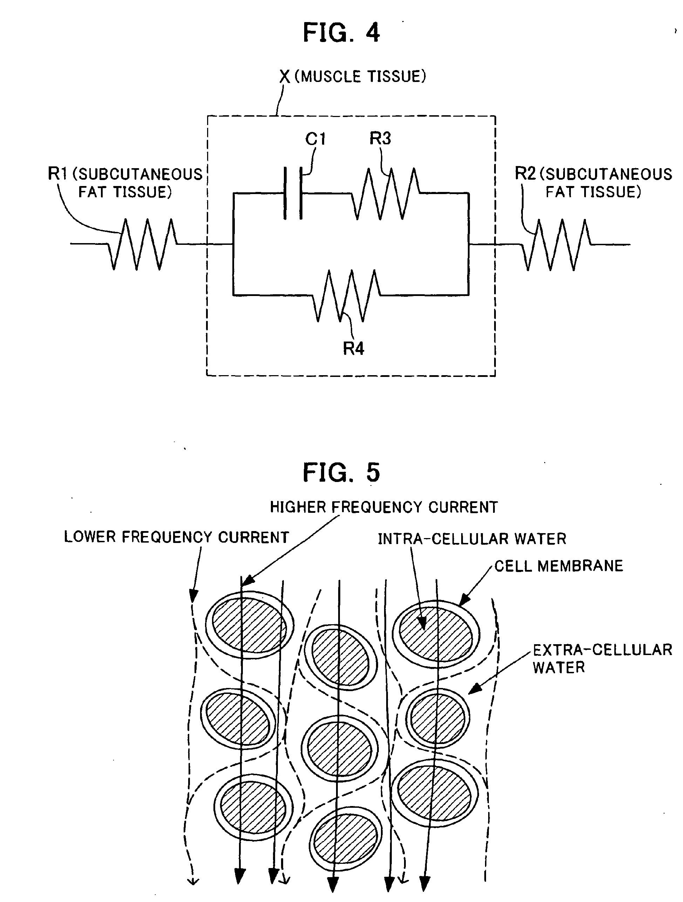

Referring now to Figs. 4 and 5, the principle of measurement of the abdomen's impedance will be described in more detail. In particular, Fig. 4 is a model view of the front surface of the abdomen of a person under test modeled by an electrical equivalent circuit of a current path therethrough; and Fig. 5 is a model view of cells illustrating different current paths depending on frequency ranges in electrolyte tissue substantially consisting of muscle tissue.

In general, it is considered, in biological tissue, that most of the fat free tissue is body water including much electrolyte through which electric current is likely to pass, but fat tissue and bone is non- electrolyte tissue including substantially no electrolyte. Accordingly, at the front surface of the abdomen, muscle tissue that is fat free tissue includes electrolyte, but subcutaneous and visceral fat that is fat tissue includes no electrolyte.

Accordingly, when the current supplying electrodes 7a and 7b are in contact with the front surface of the abdomen of the person under test then electric current is considered to flow through subcutaneous fat tissue into muscle tissue having higher electrical conductivity than visceral fat tissue. As shown in Fig. 4, the current path between the current supplying electrodes 7a and 7b is modeled in such manner that subcutaneous fat tissue under the contact positions of electrodes 7a and 7b is represented by resistors "R1" and "R2", respectively, and muscle tissue is represented by the circuit "X". In particular, the circuit "X" is an electrical equivalent circuit of electrolyte tissue wherein it is formed by a serial connection of a capacitor "C1" and a resistor "R3" in parallel with a resistor "R4".

It is noted, here, that the circuit "X" represents electrolyte tissue that is modeled at cell level. As shown in Fig. 5, the electrolyte tissue consists of cells each including intra-cellular water covered by cell membranes, and extra- cellular water outside the cells. The intra-cellular water and extra-cellular water act as resistors, and the cell membrane is considered as an insulator, but it has capacity because it is very thin. Accordingly, at a lower frequency, near DC current, the cell membrane acts as an insulator so that no current flows through the intra-cellular water. However, as the frequency of the current is increased it starts to flow through the intra-cellular water via the cell membrane. As a result, in the electrical equivalent circuit capacitor "C1"means cell membrane, resistor "R3" means intra-cellular water and resistor R4 means extra-cellular water.

In circuit "X", at the lower frequency of current used, the current only flows through resistor R4. On the other hand, at the higher frequency of current used, the current flows through the combined resistance of resistors R3 and R4 (i.e. (R3 × R4) / (R3+R4)). Because R4 < (R3 × R4) / (R3+R4), in the model of the front surface of the abdomen in Fig. 4, at lower frequency of current supplied, the impedance more strongly affected by subcutaneous fat tissue represented by resistors "R1" and "R2" would be detected, than that affected at the higher frequency of current supplied. Inversely, at the higher frequency of current supplied, the impedance more strongly affected by muscle tissue would be detected. Accordingly, as the frequency of current is increased the impedance measured becomes more strongly affected by muscle tissue.

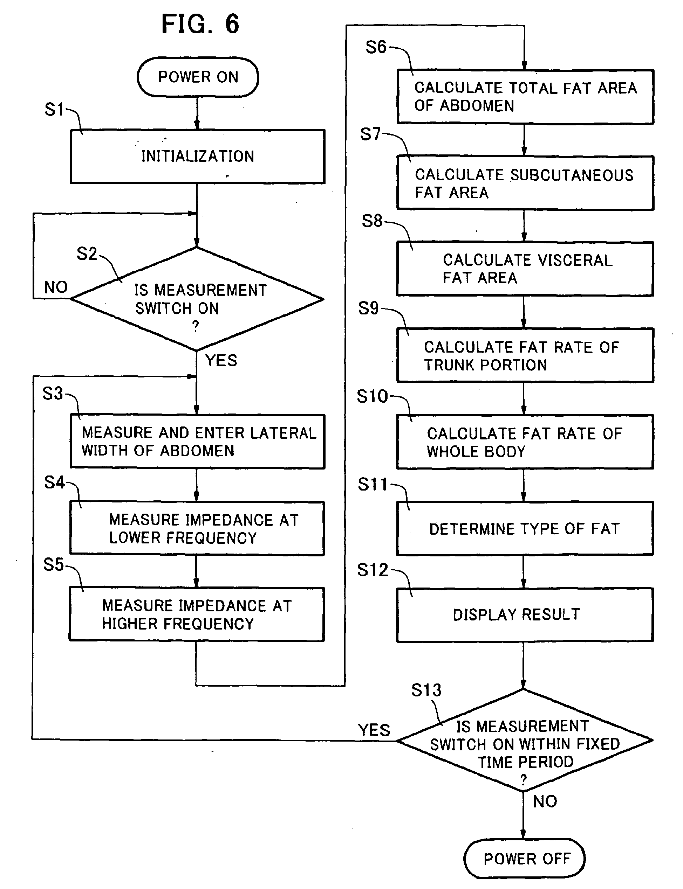

Referring now to Fig. 6 operation of the body fat measurement apparatus will be described in more detail.

First of all, the power switch 5 is pushed to turn ON the body fat measurement apparatus 1. At step S1 an initial setting of the apparatus is performed and some message is displayed on the display unit 10 informing that the measurement switch 6 can be depressed for starting measurement after the apparatus 1 is installed around a person under test. The person under test slidably draws the arm portions 4a and 4b out of the body portion 2 of the apparatus 1 to suit the lateral width of his/her abdomen, and then, lifts the arm portions at the height of his/her navel. Thereafter, the person under test presses the contact surface of the electrode support unit 3 on which the impedance measurement electrode system 9 is positioned against the front surface of his/her abdomen to complete the installation of the apparatus 1.

At step S2 it is determined whether the measurement switch 6 is depressed or not. If not, the routine proceeds to the "NO" branch and the step S2 is repeatedly performed until the measurement switch 6 is depressed, upon which the routine proceeds to the "YES" branch. Then, at step S3 the distance between the arm portions 4a and 4b drawn out of the body portion 2 is measured by the encoder unit 16 and the arithmetic unit 15 calculates the lateral width of the abdomen of the person under test based on the distance between the arm portions 4a and 4b.

At step S4 the measurement of the impedance is conducted at the predetermined lower frequency of current. The impedance value measured is temporally stored in RAM 13. At step S5 the measurement of the impedance is conducted at the predetermined higher frequency of current. The impedance value measured is temporally stored in RAM 13 in the same manner. It is assumed, here, that the lower frequency of current is 6.25 kHz, the higher frequency of current is 50 kHz, and the impedance values measured at those frequencies are represented by "$Z_{6.25}$" and "$Z_{50}$", respectively.

After completion of the impedance measurement the routine proceeds to subsequent steps wherein the index of body fat is calculated. In particular, at step S6 the total fat area of the abdomen is calculated. The controller unit 11 retrieves, from RAM 13, the impedance values measured at the lower and higher frequencies, and the lateral width of the abdomen of the person under test. In addition, the controller unit 11 retrieves, from ROM 14, a regression equation for calculating the total fat area of the abdomen, which is obtained as the result of the correlation of the total fat area of the abdomen preliminarily calculated by the CT method with said impedance values and said lateral width of abdomen. Then, the arithmetic unit 15 calculates the total fat area of the abdomen using the regression equation retrieved. The regression equation is written as follows:

$$\text{Total Fat Area of Abdomen} = p_1 + q_1 \times Z_{6.25} + r_1 \times (\text{Lateral Width of Abdomen}) + s_1 \times Z_{50} / (\text{Lateral Width of Abdomen})$$

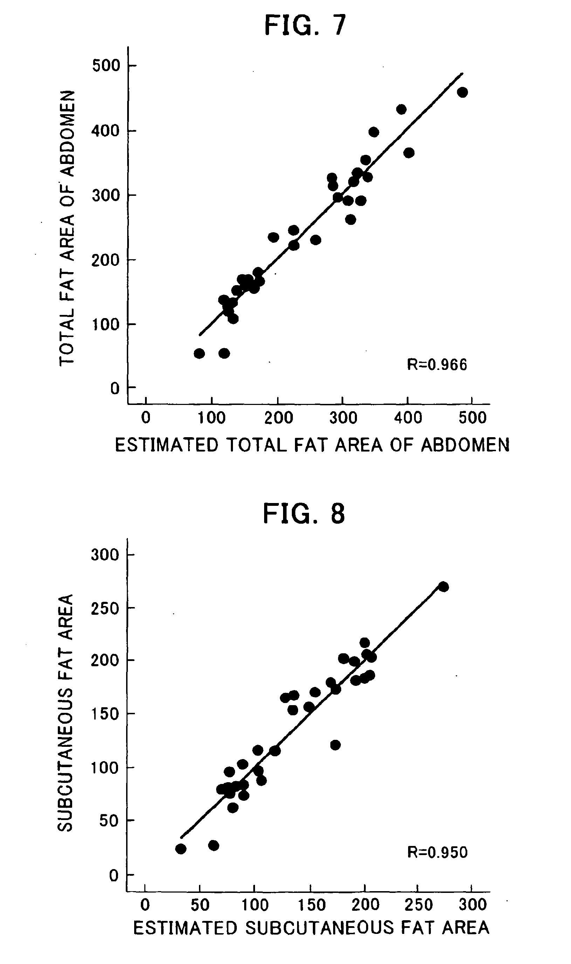

where $p_1$, $q_1$, $r_1$,and $s_1$ are constant. In this example, assuming that $p_1$ =-1500, $q_1$=80, $r_1$=-77, and $s_1$ =-960, then the total fat area of the abdomen calculated by said regression equation is highly correlated with that calculated by the CT method with a correlation factor of 0.966, as seen in a graph of Fig. 7.

Then, at step S7 the subcutaneous fat area is calculated. In the same manner as the total fat area of the abdomen as above, the subcutaneous fat area is calculated using a regression equation which is obtained as the result of the correlation of the subcutaneous fat area preliminarily calculated by the CT method with said impedance values and said lateral width of abdomen. The regression equation is written as follows:

$$\text{Subcutaneous Fat Area} = p_2 + q_2 \times Z_{6.25} + r_2 \times (\text{Lateral Width of Abdomen}) + s_2 \times Z_{50} / (\text{Lateral Width of Abdomen}),$$

where $p_2$, $q_2$, $r_2$, and $s_2$ are constant. In this example, assuming that $p_2$=-350, $q_2$=-8.0, $r_2$=35.1, and $s_2$ =500, then the subcutaneous fat area calculated by said regression equation is highly correlated with that calculated by the CT method with a correlation factor of 0.950, as seen in a graph of Fig. 8.

Then, at step S8 the visceral fat area is calculated. In the same manner as the total fat area of the abdomen and the subcutaneous fat area as above, the visceral fat area is calculated using a regression equation which is obtained as the result of the correlation of the visceral fat area preliminarily calculated by the CT method with said impedance values and said lateral width of abdomen. The regression equation is written as follows:

$$\text{Visceral Fat Area} = p_3 + q_3 \times Z_{6.25} + r_3 \times (\text{Lateral Width of Abdomen}) + s_3 \times Z_{50} / (\text{Lateral Width of Abdomen}),$$

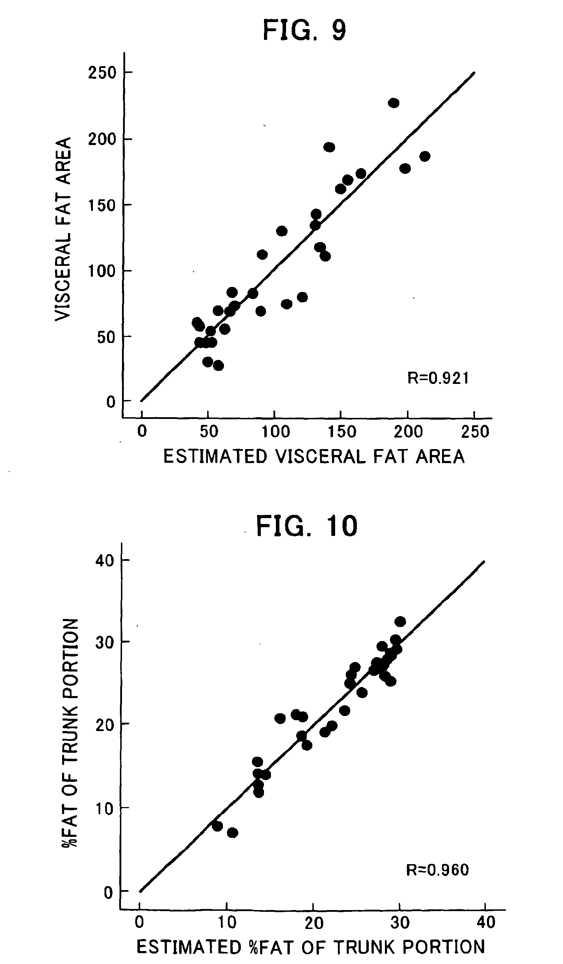

where $p_3$, $q_3$, $r_3$, and $s_3$ are constant. In this example, assuming that $p_3$= - 1030, $q_3$=90, $r_3$= - 63, and $s_3$=- 1460, then the visceral fat area calculated by said regression equation is highly correlated with that calculated by the CT method with a correlation factor of 0.921, as seen in a graph of Fig. 9.

Then, at step S9 the fat rate of the trunk portion is calculated. The fat rate of the trunk portion is calculated using a regression equation which is obtained as the result of the correlation of the fat rate of trunk portion preliminarily calculated by the DXA method with said impedance values and said lateral width of abdomen. The regression equation is written as follows:

$$\text{Fat Rate of Trunk Portion} = p_4 + q_4 \times Z_{6.25} + r_4 \times (\text{Lateral Width of Abdomen}) + s_4 \times (1/Z_{50} / (\text{Lateral Width of Abdomen})),$$

where $p_4$, $q_4$, $r_4$, and $s_4$ are constant. In this example, assuming that $p_4$=90, $q_4$=-0.99, $r_4$=-0.825, and $s_4$ =-6980, then the fat rate of the trunk portion calculated by said regression equation is highly correlated with that calculated by the DXA method with a correlation factor of 0.960, as seen in a graph of Fig. 10.

Then, at step S10 the body fat rate of whole body is calculated. In the same manner as the fat rate of the trunk portion as above, the body fat rate of whole body is calculated using a regression equation which is obtained as the result of the correlation of the body fat rate of the whole body preliminarily calculated by the DXA method with said fat rate of the trunk portion. The regression equation is written as follows:

$$\text{Body Fat Rate of Whole body} = t \times (\text{Fat Rate of Trunk Portion}) + u,$$

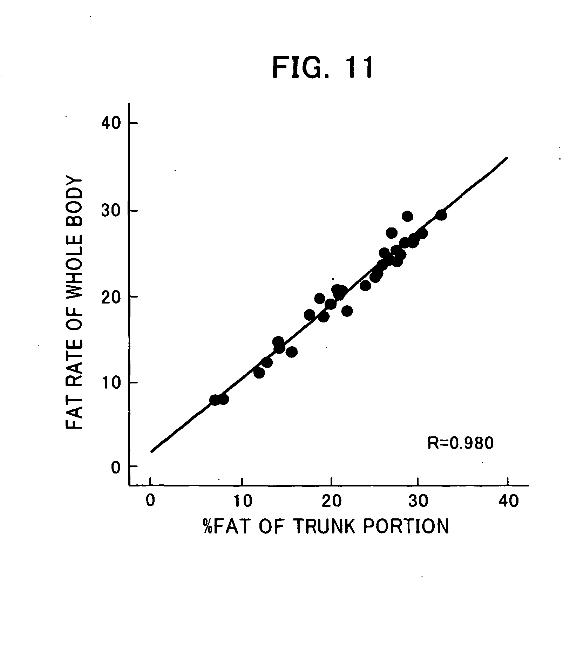

where "t" and "u" are constant. In this example, assuming that "t" =0.77 and "u" = 3.2 then the fat rate of the trunk portion is highly correlated with the body fat rate of whole body calculated by the DXA method with a correlation factor of 0.980, as seen in a graph of Fig. 11.

Due to the fact that the limbs of a person generally have less fat as compared to his/her trunk portion then the body fat rate of the whole body can be considered to mainly depend on the fat rate of the trunk portion. Accordingly, even for a person having some trouble in any of the limbs the body fat rate of the whole body can be calculated using the regression equation for calculating the body fat rate of the whole body from the fat rate of the trunk portion, in the same manner as in the case of a normal person.

At step S11 type of fat is determined depending on the difference between the subcutaneous and the visceral fat area calculated in such a manner. For example, the difference "X" is calculated as follows:

$$\text{"X"} = (\text{Subcutaneous Fat Area}) - (\text{Visceral Fat Area})$$

If "X" > 0 then the type of fat is determined to be of the subcutaneous fat type, but if "X" $\leqq 0$ then it is determined to be of the visceral fat type.

At step S12 the results of the calculation: total fat area of the abdomen, subcutaneous fat area, visceral fat area, fat rate of the trunk portion, fat rate of the whole body, and type of fat are displayed on the display unit 10. Then, at step S 13 it is determined whether the measurement switch 10 is depressed within a fixed time period while the display unit 10 continues the display. If the switch is depressed it is interpreted that the measurement is again performed. Then, the routine returns to step S3 to start the measurement. However, if the switch is not depressed within the fixed time period then the apparatus is automatically turned OFF.

It is preferable that the electrodes of the impedance measurement electrode system 9 are disposed on the front surface of the abdomen to span as wide an area as possible for widening the measurement region. In other words, preferably, the current supplying electrodes 7a and 7b are spaced apart as far as possible from each other on the front surface of abdomen so that they are disposed near both sides of abdomen. The same is true for the voltage measurement electrodes 8a and 8b. In this example, however, the distance between the electrodes of the impedance measurement electrode system 9 is substantially fixed and the dimension of the abdomen is reflected by the lateral width of abdomen. Accordingly, the distance between the current supplying electrodes 7a and 7b is preferably determined to suit a person under test having a small lateral width. In this embodiment this is determined to be 24cm.

If the distance between the voltage measurement electrodes 8a and 8b would be shorter, the measurement region, of course, becomes narrower even if the current supplying electrodes 7a and 7b are sufficiently spaced apart. Furthermore, as is well known, the distance between each current supplying electrode 7a, 7b and the adjacent voltage measurement electrodes 8a, 8b should generally be at least twice the subcutaneous fat thickness. Otherwise, the impedance measured may be affected only by subcutaneous fat tissue without inclusion of the effect of muscle tissue.

Accordingly, in this example, the distance between the current supplying electrode 7a and the voltage measurement electrode 8a, and between the current supplying electrode 7b and the voltage measurement electrode 8b is determined to be 8cm in order to suit to a person under test having thicker subcutaneous fat of 4cm.

Second Example:

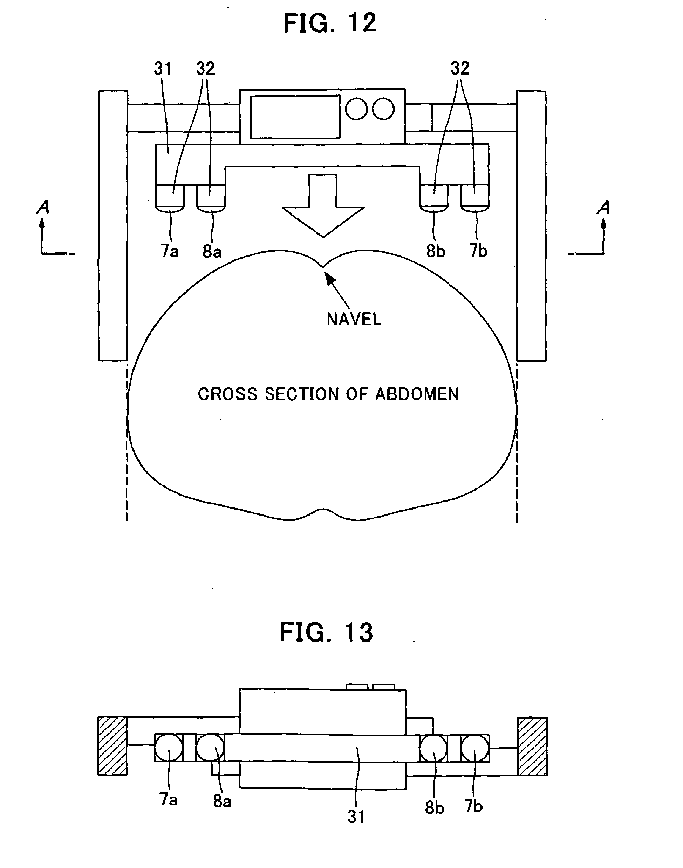

A second example is the substantially same in construction and operation as the first example as described above, except that the second example includes a different configuration of the electrode support unit and different disposition of the impedance measurement electrode system on the electrode support unit. Referring to Figs. 12 and 13, instead of the flexible electrode support unit 3 of the first example, a rigid electrode support unit 31 is provided according to the second embodiment in which a pair of current supplying electrodes 7a, 7b and a pair of voltage measurement electrodes 8a, 8b are mounted to an abdomen contact surface of the rigid electrode support unit 31 via flexible members 32 which allow said electrodes to freely change contact directions with respect to the abdomen of a person under test. In particular, a recess is formed in the rigid electrode support unit 31 between the voltage measurement electrodes 8a and 8b, which recess accommodates a rounded center portion of the front surface of the abdomen of the person under test so that a surface of the rigid electrode support unit 31 on which the electrodes 7a, 7b, 8a, 8b are mounted is directed toward the front surface of the abdomen of the person under test as the abdomen contact surface. As a result, the electrodes can closely contact the front surface of the abdomen of the person under test.

In the case where the abdomen contact surface of the rigid electrode support unit 31 can surely be made to contact the front surface of the abdomen of the person under test then the electrodes of the impedance measurement electrode system 9 may directly be mounted to the abdomen contact surface of the rigid electrode support unit 31 without any flexible members 32, which can lower the manufacturing cost of the apparatus.

Third Example:

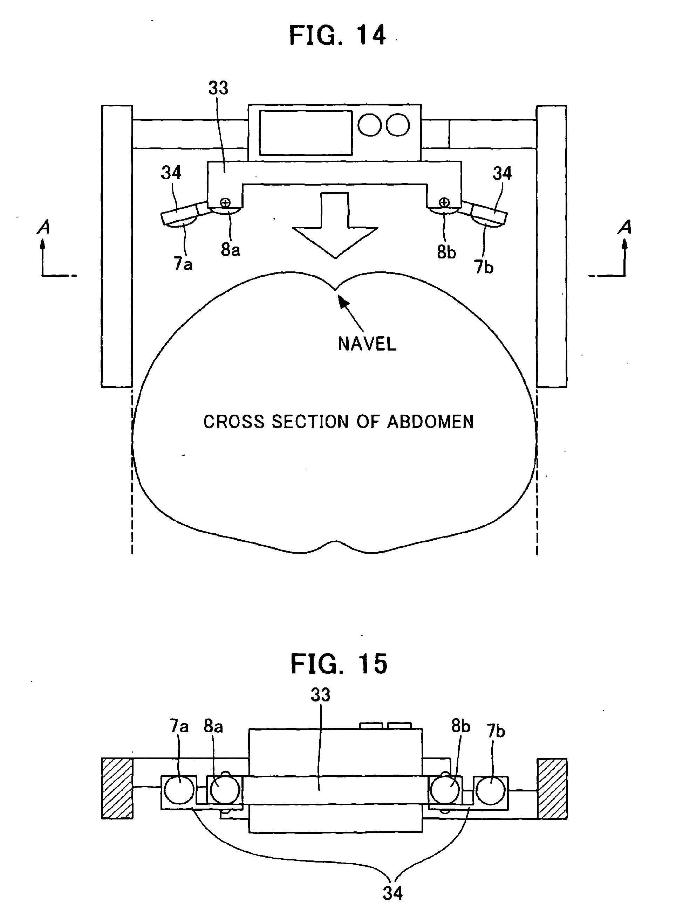

In the same manner as the second example as above, a third example is substantially the same in construction and operation as the first example, except that the third example includes a different configuration of the electrode support unit and a different disposition of the impedance measurement electrode system on the electrode support unit. Referring to Figs. 14 and 15, the apparatus comprises an electrode support unit 33 on which voltage measurement electrodes 8a and 8b are fixedly mounted. A pivot member 34 is each provided for pivotally moving about each of the voltage measurement electrodes 8a and 8b and each of the current supplying electrodes 7a and 7b is mounted to an end portion of a pivot member 34. The direction of the current supplying electrodes 7a and 7b is manually set to suit the front surface of the abdomen of a person under test.

Inversely, the current supplying electrodes 7a and 7b may fixedly be mounted while the voltage measurement electrodes 8a and 8b may be pivotally moved.

Fourth Example:

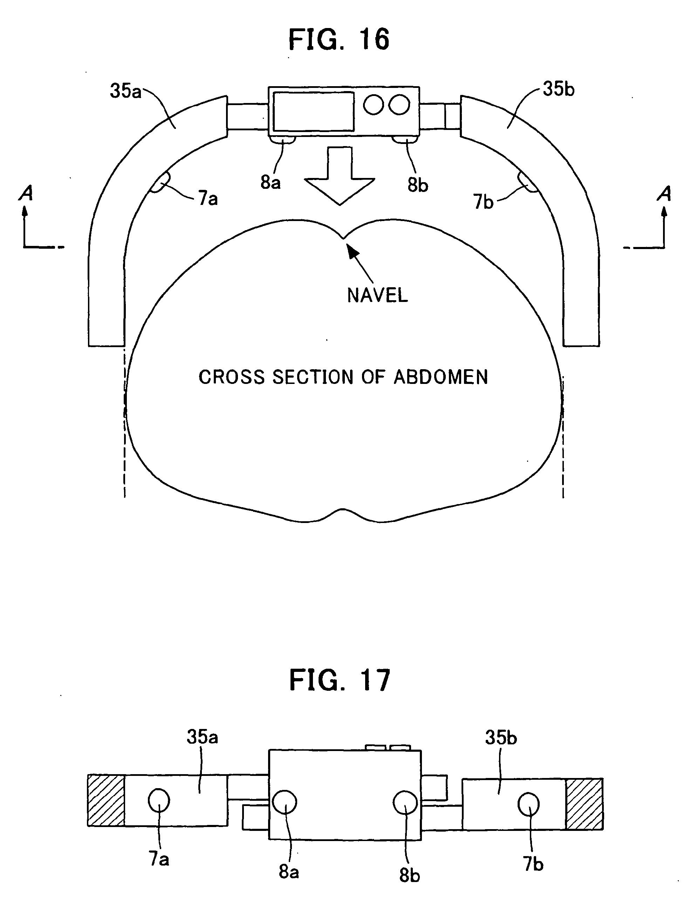

A fourth example is substantially the same in construction and operation as the first example as described above, except that the fourth example includes a different configuration of the electrode support unit and left and right arm portions as well as a different disposition of the impedance measurement electrode system on the electrode support unit. Referring to Figs. 16 and 17, electrode support arm portions 35a and 35b are formed by integrating the electrode support unit 3 with the left and right arm portions 4a, 4b in the first example. Furthermore, some sliding mechanism is included in the body portion 2, in the same manner as the left and right arm portions 4a, 4b in the first example. The electrode support arm portions 35a and 35b are curved to generally suit the shape of the front surface of the abdomen of a person under test.

In order to position the impedance measurement electrode system 9 the current supplying electrodes 7a and 7b are mounted to the curved electrode support arm portions 35a and 35b at the positions corresponding to the left and right sides of the abdomen of the person under test. The voltage measurement electrodes 8a and 8b are mounted to the abdomen contact surface of the body portion 2 at predetermined positions thereon. In this example the distance between the voltage measurement electrodes 8a and 8b is 8cm.

At measurement the electrode support arm portions 35a and 35b are slidably moved to suit the lateral width of the abdomen of the person under test, and then, they are pressed against abdomen of the person under test, together with the body portion 2, so that the impedance measurement electrode system 9 properly contacts the abdomen of the person under test.

First Embodiment:

In the first to fourth examples due to the fact that the impedance measurement region defined by the range within which the electrodes of the impedance measurement electrode system 9 can be positions is fixed in dimension relative to the lateral width of the abdomen of the person under test there may actually be variation in dimension of the measurement region depending on the different persons under test. In order to overcome this, a correction is made using the lateral width of abdomen as the correction factor so that the measurement region can be considered kept constant irrespective of the different persons under test.

On the other hand, a first embodiment of the present invention provides a body fat measurement apparatus in which the contact positions of the electrodes of an impedance measurement electrode system 9 on the abdomen of a person under test may be shifted according to the dimension of the abdomen, which obviates the measurement and the entering of the lateral width of abdomen.

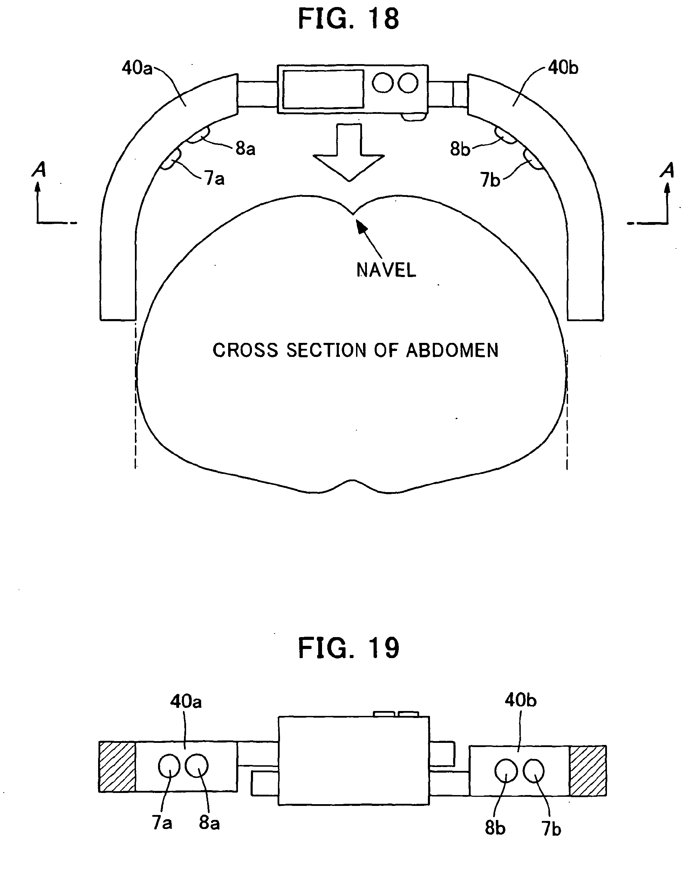

Referring now to Figs. 18 and 19, the body fat measurement apparatus according to the first embodiment will be described. The elements having the same construction and operation as those in the first to fourth examples are designated by the same reference numbers.

As shown in Fig. 18, electrode position correction arm portions 40a and 40b are curved to generally suit the shape of the front surface of the abdomen of a person under test and are supported on a sliding mechanism in a body portion 2 so that they are slidably movable along the lateral width of the abdomen, in the same manner as the electrode support arm portions 35a and 35b in the fourth example as shown in Fig. 16. As described above, the electrode support arm portions 35a and 35b are configured so that the current supplying electrodes 7a and 7b are movable, but the voltage measurement electrodes 8a and 8b are fixed. Therefore, a correction according to the lateral width of the abdomen is necessary, which is conducted in such a manner that the distance by which the arm portions have been moved is measured with an encoder 16 in the body portion 2, on the basis of which the lateral width of abdomen is automatically calculated and the correction factor is entered.

The electrode position correction arm portion 40a is provided with a current supplying electrode 7a and a voltage measurement electrode 8a. In the same manner, the electrode position correction arm portion 40b is provided with a current supplying electrode 7b and a voltage measurement electrode 8b. In this embodiment, the distance between the electrodes 7a and 8a and between the electrodes 7b and 8b is set at 8cm.

As shown in Fig. 18, at measurement the electrode position correction arm portions 40a and 40b are slidably moved to suit the lateral width of the abdomen of the person under test and are pressed to the abdomen. As the result, the electrodes of the impedance measurement electrode system 9 can contact the abdomen of the person under test at such positions that suit the dimension of the abdomen. This obviates the need of entering the lateral width of the abdomen as a correction factor.

The block diagram of the body fat measurement apparatus of this first embodiment is the same as that of the apparatus of the first example as shown in Fig. 3, except that the encoder 16 is removed in the first embodiment.

Furthermore, operation of the apparatus of the first embodiment is generally the same as that of the first example, as shown in the flow chart of Fig. 6, except that the lateral width of the abdomen is not used as the correction factor in the regression equations used for calculating the index of body fat at steps S5 to S9. The regression equation obtained as the result of the correlation of the index of body fat calculated according to the CT or the DXA method with "$Z_{50}$" and "$Z_{6.25}$" provided by the impedance measurement is retrieved from ROM 14 in the same manner as in the first example.

The determination of whether the type of fat is subcutaneous or visceral at step S11 in the flow chart of Fig 6 is performed on the basis of the difference between the subcutaneous fat area and the visceral fat area, as described above. Alternatively, in clinical treatment using the CT method. the type of fat may be determined on the basis of the ratio "V/S" where "V" is the visceral fat area and "S" is the subcutaneous fat area. In particular, the type of fat is determined to be visceral fat if "V/S">0.5, and subcutaneous fat if "V/S" $\leqq$ 0.5.

Moreover, it may be possible that the body portion 2 is provided with some input unit for entering personal data concerning the age and sex, of the person under test and the regression equations for calculating the index of body fat may be classified depending on age and sex. and stored in ROM 14. As the result, more proper regression equations can be used to produce a measurement result with higher precision.

The thickness of the subcutaneous fat can also be calculated. In general, it is well known that there is a higher correlation between the thickness of subcutaneous fat calculated according to the CT method and the impedance of the abdomen measured using the current at a frequency of 50 kHz. As described above regarding the principle of measurement of an abdomen's impedance with reference to Figs. 5 to 7, however, when using lower frequency current the impedance value is more strongly affected by subcutaneous fat tissue than when using a higher frequency current. Therefore, the regression equation obtained as the result of the correlation of the impedance at the lower frequency with the thickness of subcutaneous fat calculated by the CT method can be used to calculate the thickness of subcutaneous fat with higher precision.

Fig. 22 is a graph illustrating the correlation between the subcutaneous fat thickness and the impedance measured with a lower frequency current. A higher correlation resulted with a correlation factor of 0.903.

As described above, currents at two frequencies: a lower frequency of 6.25 kHz and a higher frequency of 50 kHz are supplied to the current supplying electrodes 7a and 7b. If the number of frequencies is increased it is possible to more precisely reflect the relationship between muscle tissue and subcutaneous fat tissue, which can increase the precision with which the measurement is conducted. Even with one frequency used impedance affected by both muscle tissue and subcutaneous fat tissue can be obtained, which provides facilitated measurement.

## Claims

1.  A body fat measurement apparatus comprising an impedance measurement electrode system (7a, 7b, 8a, 8b) including a pair of current supplying electrodes (7a, 7b) and a pair of voltage measurement electrodes (8a, 8b), those electrodes adapted for contact with a peripheral surface of the abdomen of a person under test to measure an impedance of the abdomen, on the basis of which an index of body fat of the person under test is calculated, said apparatus further comprising:

    an electrode support unit (40a, 40b);
    an electrode position shift unit; and
    an arithmetic unit, wherein
    said electrode support unit (40a, 40b) supports the impedance measurement electrode system (7a, 7b, 8a, 8b) so that the electrodes (7a, 7b, 8a, 8b) thereof are configured to contact the front surface of the abdomen of the person under test for measuring an impendance of the abdomen, and
    said arithmetic unit is configured to calculate the index of body fat of the person under test on the basis of the impedance of the abdomen;
    **characterized In that**
    said electrode position shift unit is configured to slidably move the electrode support unit (40a, 40b) along a lateral width of the abdomen allowing the electrodes (7a, 7b, 8a, 8b) of the impedance measurement electrode system (7a, 7b, 8a, 8b) to be set at suitable positions on the front surface of the abdomen;
    wherein the electrode support unit (40a, 40b) comprises a first curred electrode position correction arm portion (40a) provided with a first current supplying electrode (7a) and a first voltage measurement electrode (8a), and
    wherein the electrode support unit (40a, 40b) comprises a second curred electrode position correction arm portion (40b) provided with a second current supplying electrode (7b) and a second voltage measurement electrode (8b).

2. A body fat measurement apparatus according to claim 1 in which said electrode support unit (40a, 40b) supports the impedance measurement electrode system so that the pair of voltage measurement electrodes (8a, 8b) is positioned between the pair of current supplying electrodes (7a, 7b).

3. A body fat measurement apparatus according to claim 1 in which said electrode support unit (40a, 40b) includes an electrode direction change unit, said electrode direction change unit being configured to allow at least one of the pair of current supplying electrodes and the pair of voltage measurement electrodes to change contact directions in order to suit the shape of the abdomen.

4. A body fat measurement apparatus according to claim 1 further comprising a frequency switching unit, said frequency switching unit being configured to switch frequency of AC current supplied to the pair of current supplying electrodes in order to conduct measurements of impedance at two or more frequencies of at least one of a higher and a lower frequency range.

5. A body fat measurement apparatus according to claim 1 in which said index of body fat includes at least one of the following: total fat area of abdomen, subcutaneous fat area, subcutaneous fat thickness, visceral fat area, fat rate of trunk portion, fat rate of whole body, and ratio of subcutaneous fat to visceral fat.

**Patentansprüche**

1. Körperfett-Messvorrichtung, die ein Impedanzmess-Elektrodensystem (7a, 7b, 8a, 8b) umfasst, das ein Paar Stromzuführ-Elektroden (7a, 7b) und ein Paar Spannungsmess-Elektroden (8a, 8b) enthält, wobei diese Elektroden für Kontakt mit einer Umfangsfläche des Bauchs einer Person eingerichtet sind, die geprüft wird, um eine Impedanz des Bauchs zu messen, auf deren Basis ein Index des Körperfetts der geprüften Person berechnet wird, wobei die Vorrichtung des Weiteren umfasst:

   eine Elektroden-Trageeinheit (40a, 40b);
   eine Elektrodenpositions-Verschiebeeinheit; und
   eine Arithmetik-Einheit, wobei
   die Elektroden-Trageeinheit (40a, 40b) das Impedanzmess-Elektrodensystem (7a, 7b, 8a, 8b) so trägt, dass dessen Elektroden (7a, 7b, 8a, 8b) so eingerichtet sind, dass sie mit der Vorderseite des Bauchs der geprüften Person in Kontakt kommen, um eine Impedanz des Bauchs zu messen, und
   die Arithmetik-Einheit so konfiguriert ist, dass sie den Index des Körperfetts der geprüften Person auf Basis der Impedanz des Bauchs misst;
   **dadurch gekennzeichnet, dass**
   die Elektrodenpositions-Verschiebeeinheit so eingerichtet ist, dass sie die Elektroden-Trageeinheit (40a, 40b) in Querrichtung gleitend entlang einer Breite des Bauchs bewegt,
   und zulässt, dass die Elektroden (7a, 7b, 8a, 8b) des Impedanzmess-Elektrodensystems (7a, 7b, 8a, 8b) an geeigneten Positionen an der Vorderseite des Bauchs angeordnet werden;
   wobei die Elektroden-Trageeinheit (40a, 40b) einen ersten gekrümmten Elektrodenpositions-Korrekturarmabschnitt (40a) umfasst, der mit einer ersten Stromzuführ-Elektrode (7a) und einer ersten Spannungsmess-Elektrode (8a) versehen ist, und die Elektroden-Trageeinheit (40a, 40b) einen zweiten gekrümmten Elektrodenpositions-Korrekturarmabschnitt (40b) umfasst, der mit einer zweiten Stromzuführ-Elektrode (7b) und einer zweiten Spannungsmess-Elektrode (8b) versehen ist.

2. Körperfett-Messvorrichtung nach Anspruch 1, wobei die Elektroden-Trageeinheit (40a, 40b) das Impedanzmess-Elektrodensystem so trägt, dass die paarigen Spannungsmess-Elektroden (8a, 8b) zwischen den paarigen Stromzuführ-Elektroden (7a, 7b) positioniert sind.

3. Körperfett-Messvorrichtung nach Anspruch 1, wobei die Elektroden-Trageeinheit (40a, 40b) eine Elektrodenrichtungs-Änderungseinheit enthält, und die Elektrodenrichtungs-Änderungseinheit so eingerichtet ist, dass sie zulässt, dass sich die Kontaktrichtungen wenigstens der paarigen Stromzuführ-Elektroden oder der paarigen Spannungsmess-Elektroden so ändern, dass sie der Form des Bauchs entsprechen.

4. Körperfett-Messvorrichtung nach Anspruch 1, die des Weiteren eine Frequenz-Umschalteinheit umfasst, wobei die Frequenz-Umschalteinheit so konfiguriert ist, dass sie die Frequenz von den paarigen Stromzuführ-Elektroden zugeführtem Wechselstrom umschaltet, um Messungen der Impedanz bei zwei oder mehr Frequenzen eines höheren oder/und eines niedrigeren Frequenzbereiches durchzuführen.

5. Körperfett-Messvorrichtung nach Anspruch 1, wobei der Index des Körperfetts wenigstens eine der folgenden Größen enthält:

Gesamt-Fettfläche des Bauches, Fläche von subkutanem Fett, Dicke von subkutanem Fett, Fläche von Viszeral-Fett, Fettanteil des Rumpfabschnitts, Fettanteil des gesamten Körpers sowie Verhältnis von subkutanem Fett zu Viszeral-Fett.

**Revendications**

1. Dispositif de mesure de graisse corporelle comportant un système d'électrodes de mesure d'impédance (7a, 7b, 8a, 8b) comprenant une paire d'électrodes d'alimentation en courant (7a, 7b) et une paire d'électrodes de mesure de tension (8a, 8b), ces électrodes étant conçues pour établir un contact avec une surface périphérique de l'abdomen d'une personne soumise à un test pour mesurer une impédance de l'abdomen, sur la base de laquelle est calculé un indice de graisse corporelle de la personne soumise à un test, ledit dispositif comportant en outre :

une unité de support d'électrodes (40a, 40b) ;
une unité de déplacement de position d'électrode ; et
une unité arithmétique, dans lequel
ladite unité de support d'électrodes (40a, 40b) supporte le système d'électrodes de mesure d'impédance (7a, 7b, 8a, 8b) de telle sorte que les électrodes (7a, 7b, 8a, 8b) dudit système soient configurées pour établir un contact avec la surface avant de l'abdomen de la personne soumise à un test pour mesurer une impédance de l'abdomen, et
ladite unité arithmétique est configurée pour calculer l'indice de graisse corporelle de la personne soumise à un test en se basant sur l'impédance de l'abdomen ;
**caractérisé en ce que**
ladite unité de déplacement de position d'électrode est conçue pour déplacer par glissement l'unité de support d'électrodes (40a, 40b) selon une direction latérale de la largeur de l'abdomen en permettant le positionnement des électrodes (7a, 7b, 8a, 8b) du système d'électrodes de mesure d'impédance (7a, 7b, 8a, 8b) dans des positions appropriées sur la surface avant de l'abdomen ;
dans lequel l'unité de support d'électrodes (40a, 40b) comporte une première partie courbée de bras de correction de position d'électrode (40a) comportant une première électrode d'alimentation en courant (7a) et une première électrode de mesure de tension (8a), et dans lequel l'unité de support d'électrode comporte une deuxième partie courbée de bras de correction de position d'électrode (40b) munie d'une deuxième électrode d'alimentation en courant (7b) et d'une deuxième électrode de mesure de tension (8b).

2. Dispositif de mesure de graisse corporelle selon la revendication 1, dans lequel ladite unité de support d'électrodes (40a, 40b) supporte le système d'électrodes de mesure d'impédance de telle sorte que la paire d'électrodes de mesure de tension (8a, 8b) soit positionnée entre la paire d'électrodes d'alimentation en courant (7a, 7b).

3. Dispositif de mesure de graisse corporelle selon la revendication 1, dans lequel ladite unité de support d'électrodes (40a, 40b) comporte une unité de changement de direction d'électrode, ladite unité de changement de direction d'électrode étant configurée pour permettre à l'une au moins de la paire d'électrodes d'alimentation en courant et de la paire d'électrodes de mesure de tension de changer la direction de contact afin de s'adapter à la forme de l'abdomen.

4. Dispositif de mesure de graisse corporelle selon la revendication 1, comportant en outre une unité de commutation de fréquences, ladite unité de commutation de fréquences étant configurée pour commuter la fréquence de courant alternatif alimentant la paire d'électrodes d'alimentation en courant afin de procéder à des mesures d'impédance selon deux fréquences, au plus, appartenant à au moins un intervalle parmi des intervalles supérieur et inférieur.

5. Dispositif de mesure de graisse corporelle selon la revendication 1, dans lequel ledit indice de graisse corporelle comprend au moins l'une des valeurs suivantes : surface de graisse total de l'abdomen, surface de graisse sous-cutanée, épaisseur de graisse sous-cutanée, surface de graisse viscérale, taux de graisse du tronc, taux de graisse corporelle et rapport de la graisse sous-cutanée à la graisse viscérale.

# FIG. 1

1

4a  3  2  10  5  6  4b

A

7a  8a  8b  7b

NAVEL

CROSS SECTION OF ABDOMEN

A

# FIG. 2

2  5  6

4a  3  4b

7a  8a  8b  7b

# FIG. 3

# FIG. 4

X (MUSCLE TISSUE)

R1 (SUBCUTANEOUS FAT TISSUE)

C1    R3

R4

R2 (SUBCUTANEOUS FAT TISSUE)

# FIG. 5

HIGHER FREQUENCY CURRENT

LOWER FREQUENCY CURRENT

INTRA-CELLULAR WATER

CELL MEMBRANE

EXTRA-CELLULAR WATER

# FIG. 6

POWER ON

S1 — INITIALIZATION

S2 — IS MEASUREMENT SWITCH ON ?

NO

YES

S3 — MEASURE AND ENTER LATERAL WIDTH OF ABDOMEN

S4 — MEASURE IMPEDANCE AT LOWER FREQUENCY

S5 — MEASURE IMPEDANCE AT HIGHER FREQUENCY

S6 — CALCULATE TOTAL FAT AREA OF ABDOMEN

S7 — CALCULATE SUBCUTANEOUS FAT AREA

S8 — CALCULATE VISCERAL FAT AREA

S9 — CALCULATE FAT RATE OF TRUNK PORTION

S10 — CALCULATE FAT RATE OF WHOLE BODY

S11 — DETERMINE TYPE OF FAT

S12 — DISPLAY RESULT

S13 — IS MEASUREMENT SWITCH ON WITHIN FIXED TIME PERIOD ?

YES

NO

POWER OFF

17

## FIG. 7

## FIG. 8

## FIG. 9

## FIG. 10

# FIG. 11

R=0.980

FAT RATE OF WHOLE BODY

%FAT OF TRUNK PORTION

# FIG. 12

CROSS SECTION OF ABDOMEN

NAVEL

# FIG. 13

## FIG. 14

NAVEL

CROSS SECTION OF ABDOMEN

## FIG. 15

# FIG. 16

NAVEL

CROSS SECTION OF ABDOMEN

# FIG. 17

## FIG. 18

40a

40b

8a

8b

7a

7b

A

A

NAVEL

CROSS SECTION OF ABDOMEN

## FIG. 19

40a

40b

7a    8a

8b    7b

FIG. 20

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1269917 A1

- JP 3396674 B